# EUROPEAN PATENT SPECIFICATION

(11) **EP 3 642 359 B1**
(45) Date of publication and mention of the grant of the patent: **30.03.2022**
(21) Application number: 17768251.5
(22) Date of filing: 20.06.2017
(51) Int. Cl.: C12Q 1/6816, C12Q 1/68, C12Q 1/6851

(54) **METHODS FOR DNA TARGETS DETECTION DIRECTLY IN CRUDE SAMPLES THROUGH POLYMERASE CHAIN REACTION AND GENOTYPING VIA HIGH RESOLUTION MELTING ANALYSIS**
VERFAHREN ZUR DIREKTEN DETEKTION VON DNA-ZIELEN IN ROHPROBEN DURCH POLYMERASEKETTENREAKTION UND GENOTYPISIERUNG MITTELS HOCHAUFLÖSENDER SCHMELZANALYSE
PROCÉDÉS POUR LA DÉTECTION D'ADN CIBLES DIRECTEMENT DANS DES ÉCHANTILLONS BRUTS PAR RÉACTION EN CHAÎNE PAR POLYMÉRASE ET GÉNOTYPAGE PAR LE BIAIS D'UNE ANALYSE DE FUSION À HAUTE RÉSOLUTION

(43) Date of publication of application: 29.04.2020
(73) Proprietor: ULISSE BIOMED S.P.A., 33100 Udine (IT)
(72) Inventor: IPPODRINO, Rudy, 34135 Trieste (TS) (IT); MARINI, Bruna, 34136 Trieste (TS) (IT); AVIAN, Alice, 34136 Trieste (TS) (IT); MOCENIGO, Marco, 34144 Trieste (TS) (IT); FOSCHI, Nicola, 47121 Forli(FC) (IT); MAURO, Elisabetta, 30028 San Michele al Tagliamento (VE) (IT)
(74) Representative: Petraz, Gilberto Luigi
(86) International application number: PCT/IT2017/000126
(87) International publication number: WO 2018/235110

(56) References cited:
- WO-A1-2014/029851
- US-B1- 8 980 562
- TA-HSIEN LEE ET AL: "High-Resolution Melting Molecular Signatures for Rapid Identification of Human Papillomavirus Genotypes", PLOS ONE, vol. 7, no. 8, 20 August 2012 (2012-08-20) , page e42051, XP055083323, DOI: 10.1371/journal.pone.0042051
- Y. LIAO ET AL: "Simultaneous Detection, Genotyping, and Quantification of Human Papillomaviruses by Multicolor Real-Time PCR and Melting Curve Analysis", JOURNAL OF CLINICAL MICROBIOLOGY, vol. 51, no. 2, 1 February 2013 (2013-02-01), pages 429-435, XP055448223, US ISSN: 0095-1137, DOI: 10.1128/JCM.02115-12
- ELSA HERRAEZ-HERNANDEZ ET AL: "HPV Direct Flow CHIP: A new human papillomavirus genotyping method based on direct PCR from crude-cell extracts", JOURNAL OF VIROLOGICAL METHODS, vol. 193, no. 1, 1 October 2013 (2013-10-01), pages 9-17, XP055447844, NL ISSN: 0166-0934, DOI: 10.1016/j.jviromet.2013.04.018
- LEE SIN HANG ET AL: "Molecular tests for human papillomavirus (HPV), Chlamydia trachomatis and Neisseria gonorrhoeae in liquid-based cytology specimen", BMC WOMEN'S HEALTH, BIOMED CENTRAL, LONDON, GB, vol. 9, no. 1, 9 April 2009 (2009-04-09), page 8, XP021049617, ISSN: 1472-6874, DOI: 10.1186/1472-6874-9-8
- LEE SIN HANG ET AL: "Routine human papillomavirus genotyping by DNA sequencing in community hospital laboratories", INFECTIOUS AGENTS AND CANCER, BIOMED CENTRAL LTD, LO, vol. 2, no. 1, 5 June 2007 (2007-06-05), page 11, XP021030886, ISSN: 1750-9378, DOI: 10.1186/1750-9378-2-11

## Description

### FIELD OF THE INVENTION

Embodiments of the present disclosure relate to methods for DNA targets detection directly in crude samples through PCR and genotyping via HRM analysis. In particular, embodiments of the present disclosure describe methods to detect several pathogenic strains responsible for sexually transmitted diseases, such as, but not limited to HR-HPV (High Risk Human Papilloma Virus).

More in details, the present disclosure allows, in one multiplex assay, the detection of pathogenic DNA directly from crude biological specimens, including for instance vaginal or cervical mucus, without a DNA extraction step.

### BACKGROUND OF THE INVENTION

Polymerase Chain Reaction (PCR) is a robust technique used for diagnostic applications worldwide. PCR consists of a primer extension reaction that amplifies specific nucleic acids in vitro. The reaction exploits a thermostable DNA polymerase and it is based on several cycles including different temperature steps that allow DNA denaturation, primer annealing and polymerase-mediated elongation of the target DNA.

PCR can be combined with fluorescent dyes able to intercalate with double stranded DNA or fluorescently-labelled DNA probes; in this way, the signal derived from newly synthesized amplicons can be quantitatively acquired in real-time through fluorescence acquisition.

High Resolution Melting (HRM) is an additional post-PCR step that further characterizes the amplicons by studying thermal denaturation of double-stranded DNA. This occurs through the analysis of amplicon disassociation (melting) behaviour in a ramp of temperatures usually ranging from 65°C to 95°C, with a fluorescence acquisition rating of each 0.1 °C/sec or less.

HRM is used in diagnostics, for example in the context of genetic tests able to identify SNPs in polymorphic alleles and it has been proposed for a variety of applications including pathogen detection.

Nowadays HRM analysis requires highly pure extracted DNA: this restricts its application mostly to high-income settings where DNA extraction is automated. In particular, HRM requires highly pure extracted DNA because DNA prep contaminations cause unpredictable distortions of the melting curves.

Moreover, to achieve high specificity, HRM is performed in PCR reactions requiring target-dedicated primers and probe sets, that are not usually a quite affordable reagent. Nevertheless, HRM has an enormous unexplored potential for useful applications also in low-income settings, such as the characterization of human pathogens.

Indeed, there is a felt need to provide methods that can be used to apply HRM technique directly in crude samples, without the requirement of expensive and time-consuming DNA extraction steps, and without the use of expensive primers and probe pairs.

It is well known that HPV is a necessary cause of invasive cervical cancer worldwide.

This tumour is one of the most frequent tumour in women with more than 500.000 cases worldwide¹ and it is mostly determined by persistent infection of high risk HPV (HR-HPV) genotypes; approximately 20 cancer-associated HR-HPV have been described so far, being HPV16 and HPV18 the most frequent (70% of all invasive cervical cancer)^{2,3}. Effective cervical cancer screening programs have reduced the incidence and the mortality of this tumor⁴; initially screening was performed with the Papanicolau test (Pap test), a cytological test that allows to identify precancerous cells after collection of cervical specimen by endocervical brush, and after plating and staining the specimen on a microscopic slide.

This technique shows a very high positive predictive value but it has poor average sensitivity⁵ and low negative predictive value.

Moreover, the use of endocervical brush can be painful and invasive, and this psychologically inhibits the access to the test by part of the population, especially in contexts where for social and religious reasons, women rarely undergo gynaecological visits.

Current studies indicate that HPV DNA testing is more efficient for the prevention of invasive cervical cancer than the Pap test screening alone, given the higher negative predictive value of the HPV DNA test⁶⁻⁸. In addition, given the variability of positive predictive values for cancer development among different HR-HPV genotypes, HPV genotyping is useful for the triage of HPV-positive patients, both in cervical cancer and in other HPV-related cancers (such as ano-genital and oropharyngeal tumours)⁹⁻¹³. It might as well discriminate the transient infections from the persistent ones, being the latter the true risk factor for cancer transformation. Finally, genotyping test might represent an important tool for vaccine monitoring and herd immunity in population study, to assess the variation of the prevalence of each genotype in vaccinated and notvaccinated population¹⁴.

Nowadays the most diffused tests can be classified into two groups¹⁵:
1. Signal amplification assays, such as the Digene ^{®} HPV test based on Hybrid Capture^{®} 2 (HC2, Qiagen) technologies and the Cervista ^{®} HPVHR assay. The HC2 is based on a non-radioactive signal-amplification method based on the hybridization of 13 labelled HR-HPV-specific RNA probe sets to the target HPV DNA; the DNA-RNA hybrid is recognized by a specific antibody conjugated to alkaline phosphatase and adsorbed to the bottom of microwells. Enzyme activity, measured through a chemiluminescent signal, is directly proportional to the amount of target DNA. Cervista ^{®} HPV-HR assay exploits a fluorescenceresonance energy transfer (FRET)-based technology in which there are two probes for each HPV type; it can detect 14 HR-HPV types.
   These techniques are quantitative and have a low false-positive rate but they do not provide any HPV genotyping information and they require dedicated instruments.
2. Nucleic Acid Amplification Assays, based on Real Time PCR method; among these tests, COBAS 4800 HPV test (by Roche) is the most diffused and exploits several primers and probe pairs in a fully automated system, providing genotyping information for HPV16 and HPV18. This test, as the Qiagen's, is considered the golden standard, characterized by a very high sensitivity.

Other PCR-based tests include the Papillocheck^{®} (Greiner Bio-One) capable of detecting and genotyping 24 HPV types with 28 probes spotted on a DNA chip; after PCR the hybridization is performed on a microarray chip that is automatically scanned and analysed.

The Linear Array HPV Genotyping (Roche) is a PCR-based assay coupled with reverse line blot hybridization, able to discriminate 37 HPV types; however, the test is quite time-consuming and it can provide equivocal results also due to easy cross-hybridization. Cepheid HPV is the only test present on the market able to use directly PreservCyt samples in a ready-to-use cartridge that then undergoes a dedicated PCR.

All these tests require specific instruments and are not affordable enough for a widespread diffusion, especially in developing countries.

PCR multiplexing is often a tricky analysis due to the use of several primer sets that lead to fluorescence aspecific amplification signals (such as background derived from primer dimers formation). This can be limited thanks to fluorescently-labelled probes (e. g. Taqman probes, Life Technologies), that ensure a higher specificity compared to intercalating dyes that do not make distinction between amplicons. However, fluorescently-labelled probes are more expensive, affect the test robustness and the analysis requires more than one fluorescence detector.

There is a need to improve methods for DNA targets detection through PCR and genotyping via HRM, which overcome at least one of the drawbacks in the art.

In particular, one aim of the present disclosure is to provide methods for DNA targets detection directly from crude samples through PCE and genotyping via HRM analysis, while improving significantly the robustness of the PCR performance.

One further aim of the present disclosure is to provide methods that can work directly using crude samples such as, for instance, vaginal and cervical mucus, thus providing a test that has a better compliance with respect to the women population.

Still one further aim of the present disclosure is to avoid DNA extraction while improving significantly the robustness of the HRM performance.

US 8 980 562 B1 (MANNA PRADIP [US]; 2015-03-17) discloses a method of genotyping HPV in patient samples comprising extracting DNA from the sample using a buffer containing protein-digesting enzymes, PCR multiplex amplification using two or more primer pairs and melting curve analysis using intercalating agents in real time.

LEE SIN HANG ET AL, 2009 ("Molecular tests for human papillomavirus (HPV), Chlamydia trachomatis and Neisseria gonorrhoeae in liquid-based cytology specimen", BMC WOMEN'S HEALTH, BIOMED CENTRAL, vol. 9, no. 1, page 8) discloses a method of DNA target detection (HPV) directly in crude samples (liquid-based Pap cytology specimens) comprising diluting said crude sample in proteinase-K solution. The method also includes a PCR reaction including two primer pairs (nested PCR), which involves a temperature ramp, and sequencing of the amplification products for HPV genotyping.

### SUMMARY OF THE INVENTION

According to embodiments, a method for DNA targets detection directly in crude samples through PCR and genotyping via HRM analysis is provided. According to one embodiment, the invention provides a method for pathogen DNA targets detection directly in crude samples through Polymerase Chain Reaction (PCR) and genotyping via High Resolution Melting (HRM) analysis, said pathogen being human papilloma virus (HPV), said method comprising:- providing a crude sample to be subjected to detection analysis;- diluting said crude sample using a processing buffer comprising a proteindigesting enzyme, wherein the crude sample is diluted up to a protein concentration ranging from 0.1f xg/f xl to 10µg/µl, wherein said protein-digesting enzyme is proteinase K and is comprised between 1 mg/mL and 1[j, g/mL in said processing buffer, said processing buffer comprising TrisHCl buffer solution, said TrisHCl buffer solution being comprised between 1 M and 10 mM and the pH being between 7.0 and 10.0;- treating the diluted crude sample by performing a ramp of increasing and then decreasing temperature;- providing a PCR reaction mixture comprising two or more pairs of amplification primers for amplifying in a multiplex approach two or more target nucleic acids and an amplification buffer comprising an intercalating molecule or compound incorporated into the double-stranded amplicon and emitting a detectable signal;- performing PCR amplification using said PCR reaction mixture and said treated diluted crude sample;- performing, at the end of the PCR amplification, an HRM analysis on the PCR reaction mixture and said treated diluted crude sample previously subjected to PCR amplification; wherein the method further comprises monitoring, during the HRM analysis, the change in the signal emission resulting from the temperature-induced denaturation of the double-stranded amplicons into two single-stranded DNA, due to the release of the intercalating molecule or compound, wherein the method further comprises detecting DNA targets in the crude sample, through a reader analysing the signal variation and obtaining the result of the analysis through a graphic interface connected to said reader, wherein treating the diluted crude sample by performing a ramp of increasing and then decreasing temperature comprises performing the following temperature steps:- a first incubation step ranging from 5 to 60 minutes in a temperature range between 25°C and 70°C,- a second incubation step ranging from 5 to 60 minutes in a temperature range between 90°C and 100°C,- a third incubation step ranging from 5 to 60 minutes in a temperature range between 0°C and 10°C.

According to further embodiments, the invention provides a diagnostic kit for pathogen DNA targets detection directly in crude samples through Polymerase Chain Reaction (PCR) and genotyping via High Resolution Melting (HRM) analysis, said pathogen being human papilloma virus (HPV), wherein said kit comprises a processing buffer and a Polymerase Chain Reaction (PCR) reaction mixture used for performing PCR amplification and a subsequent HRM analysis on the PCR reaction mixture previously subjected to real-time PCR, wherein said processing buffer comprises a protein-digesting enzyme, wherein said protein-digesting enzyme is proteinase K and is comprised between 1 mg/mL and 1[j, g/mL in said processing buffer, said processing buffer comprising TrisHCl buffer solution, said TrisHCl buffer solution being comprised between 1 M and 10 mM and the pH being between 7.0 and 10.0, and further wherein said PCR reaction mixture comprises two or more pairs of amplification primers for amplifying in a multiplex approach two or more target nucleic acids and an amplification buffer comprising an intercalating molecule or compound incorporated into the double-stranded amplicon and emitting a detectable signal.

In a further embodiment, the invention provides an apparatus for performing pathogen DNA targets detection directly in crude samples through Polymerase Chain Reaction (PCR) and genotyping via HRM analysis, said pathogen being human papilloma virus (HPV), wherein said apparatus comprises:- a processing buffer comprising a protein-digesting enzyme, for diluting said crude sample, wherein said protein-digesting enzyme is proteinase K and is comprised between 1 mg/mL and 1[j, g/mL in said processing buffer, said processing buffer comprising TrisHCl buffer solution, said TrisHCl buffer solution being comprised between 1 M and 10 mM and the pH being between 7.0 and 10.0;- a heating/cooling device configured for subjecting the diluted crude sample to a treatment according to a ramp of increasing and then decreasing temperature, comprising performing the following temperature steps:- a first incubation step ranging from 5 to 60 minutes in a temperature range between 25°C and 70°C,- a second incubation step ranging from 5 to 60 minutes in a temperature range between 90°C and 100°C,- a third incubation step ranging from 5 to 60 minutes in a temperature range between 0°C and 10°C;- a PCR reaction mixture comprising two or more pairs of amplification primers to amplify through multiplex approach two or more nucleic acids targets and an amplification buffer comprising an intercalating molecule or compound incorporated into the double-stranded amplicon and emitting a detectable signal;- a PCR amplification device configured for using said PCR reaction mixture and for subjecting the treated diluted crude sample to a PCR amplification;- a device for performing an HRM analysis on the PCR reaction mixture previously subjected to PCR amplification; wherein the PCR reaction mixture comprises two or more pairs of amplification primers for amplifying in a multiplex approach two or more target nucleic acids;- monitoring means for monitoring, during the HRM analysis, the change in the signal emission resulting from the temperature-induced denaturation of the double-stranded amplicons into two single-stranded DNA, due to the release of the intercalating molecule or compound,- a reader analysing the signal variation for detecting DNA targets in the crude sample, so that the result of the analysis can be obtained through a graphic interface connected to said reader.

In advantageous embodiments, the methods according to the present disclosure have been applied to successfully improve the detection of HPV DNA, the main cause of cervical cancer.

According to the present disclosure, the methods described herein can work using for instance vaginal and cervical mucus, a biological sample that women can easily selfcollect with no invasiveness and no pain, instead of endocervical brush that can be painful and invasive, thus psychologically inhibiting the access to the test by part of women population.

Moreover, the methods described herein work directly on crude samples: after a brief pre-treatment, the crude sample, e.g. vaginal or cervical mucus, is directly loaded in the reaction buffer. No DNA extraction is required, in contrast to the tests known in the prior art. The sample can be, for instance, vaginal or cervical mucus but also the common PreservCyt, ThinPrep (Hologic) or other unprocessed crude samples.

The DNA target is a HPV pathogen DNA target.

Moreover, according to the present disclosure, an innovative, fast and affordable multiplexing technology can be provided, enabling direct PCR detection, in particular real-time direct PCR, of multiple pathogenic, e.g. viral, DNA targets in a single reaction, using specific reaction mixture and conditions.

Embodiments described herein allow to detect, for example, HPV genotypes through a specific HRM analysis, by directly analysing crude samples.

Advantageously, embodiments described herein allow to detect up to 200 HPV types, Alpha-HPV, Beta-HPV, Gamma-HPV, Mu-HPV, Nupapillomavirus including the high risk and low risk HPV types 6, 11, 16, 18, 26, 31, 33, 35, 39, 40, 42, 45, 51, 52, 53, 54, 55, 56, 58, 59, 61, 62, 64, 66, 67, 68, 69, 70, 71, 72, 73 (MM9), 81, 82 (MM4), 83 (MM7), 84 (MM8), IS39, CP6108.

The viral genome amplification reaction can be coupled to the amplification of a DNA loading control target (human beta-globin gene for example). Advantageously, the methods according to the present disclosure can be performed with the most common real time PCR machines present on the market.

Other pathogens that can be detect according to the present disclosure include for instance other pathogens than HPV responsible for sexually transmissible diseases, such as the bacterium *Treponema pallidum* subspecies pallidum, responsible for the syphilis infection, the bacterium *Neisseria gonorrhoeae,* responsible for the gonorrhoea infection, the bacterium *Chlamydia Trachomatis,* responsible for the Chlamydia infection, and the integrated HIV1 and HIV2 viral DNA.

Embodiments described herein according to the present disclosure fully solve the above-mentioned issues of the tests and methods of the prior art, and further provide at least the following advantages:
- direct test: embodiments of the present disclosure describe a method to analyse and characterize DNA targets without DNA extraction steps, but directly in preprocessed crude samples, such as cervical and vaginal mucus, and other bodily fluids such as saliva, blood, urine, biopsies, formalin-fixed paraffin-embedded (*FFPE*) tissue cells, fine needle aspiration biopsies or similar biological samples;
- affordability: embodiments of the present disclosure are affordable compared to the afore-mentioned tests and methods of the prior art, because they do not use dozens of expensive labelled-probes but a unique intercalating molecule or compound, e.g. an intercalating dye;
- open-accessibility and versatility: embodiments of the present disclosure can be performed by any real-time PCR machine and do not require tailored and customized instruments;
- single-channel: result of embodiments of the present disclosure can be provided through the analysis of a single signal, e.g. fluorescence, channel, in contrast to the afore-mentioned tests and methods of the prior art, where more than one channel is used. The selected channel can be chosen among those embedded in any real-time PCR machine and this makes embodiments of the present disclosure suitable for any real-time PCR machine.

These and other features, aspects and advantages of the present disclosure will become better understood with reference to the following description, the drawings and appended claims. The drawings, which are incorporated in and constitute a part of this specification, are used to illustrate embodiments of the present subject matter and, together with the description, serve to explain the principles of the disclosure.

### BRIEF DESCRIPTION OF THE DRAWINGS

So that the manner in which the above recited features of the present invention can be understood in detail, a more particular description of the invention, briefly summarized above, may be had by reference to embodiments. The accompanying drawings relate to embodiments of the disclosure and are described in the following:
- Figure 1 is a graph showing amplification plots of PCR amplification reaction of sample treated with processing buffer according to the present disclosure (black curve) and non-treated samples (grey curve);
- Figure 2 is a graph showing melting curve plots of HRM genotyping analysis of sample treated according to the present disclosure (black curve) and non-treated samples (grey curve);
- Figure 3 shows the results of SDS-PAGE gel analysis followed by Coomassie staining of treated sample (lane 1) vs untreated sample (lane 2);
- Figure 4 is a graph showing amplification plots of PCR amplification reaction of diluted crude sample according to the present disclosure (black curve) and non-diluted sample (grey curve);
- Figure 5 is a graph showing melting curve plots of HRM genotyping analysis of diluted crude sample according to the present disclosure (grey curve) and non-diluted sample (black curve);
- Figure 6 is a graph showing amplification plots of PCR amplification reaction using a processing buffer with endoprotease according to embodiments described herein (black curve) and without endoprotease (grey curve);
- Figure 7 is a graph showing melting curve plots of HRM genotyping analysis using a processing buffer with endoprotease according to embodiments described herein (black curve) and without endoprotease (grey curve);
- Figure 8 is a graph showing amplification plots of PCR amplification reaction using additives according to embodiments described herein (black curve) and without additives (grey curve);
- Figure 9 is a graph showing melting curve plots of HRM genotyping analysis obtained by using a crude sample treated through the processing buffer according to the present disclosure (black curve) followed by the amplification through the PCR reaction mixture and by using a crude sample loaded into a standard PCR reaction mixture, without the treatment with the processing buffer according to the present disclosure (grey curve);
- Figures 10 schematically show respectively a container including first phase and second phase and steps of a combined method using the container including first phase and second phase, according to embodiments described herein.

### DETAILED DESCRIPTION OF THE EMBODIMENTS

Reference will now be made in detail to the various embodiments of the invention, using the attached figures. Generally, only the differences with respect to individual embodiments are described. Each example is provided by way of explanation of the invention and is not meant as a limitation of the invention.

All the percentages and ratios indicated refer to the weight of the total composition (for example indicated as % w/w), unless otherwise indicated. All the measurements are made, unless otherwise indicated, at 25°C and atmospheric pressure. All the temperatures, unless otherwise indicated, are expressed in degrees Centigrade.

All the ranges reported here shall be understood to include the extremes, including those that report an interval "between" two values. Furthermore, all the ranges reported here shall be understood to include and describe the punctual values included therein, as well as all the sub-intervals. Moreover, all the ranges are intended as such that the sum of the values comprised therein, in the final composition, gives 100%, in particular considering that the person of skill will know how to choose the values of the ranges so that the sum does not exceed 100%.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

Embodiments of the present disclosure generally relate to a method that uses a processing buffer for pre-treating and diluting a crude sample prior to subjecting it to direct PCR amplification and HRM analysis.

Advantageously, the sample can be can be vaginal or cervical mucus, other bodily fluids, saliva, blood, urine, biopsies, formalin-fixed paraffin-embedded (*FFPE*) tissue cells, fine needle aspiration biopsies or similar biological samples.

The method of the present disclosure is able to detect DNA targets directly in crude samples through PCR and genotyping via HRM analysis.

According to the invention, it is provided a method for pathogen DNA targets detection directly in crude samples through Polymerase Chain Reaction (PCR) and genotyping via High Resolution Melting (HRM) analysis, said pathogen being human papilloma virus (HPV), said method comprising:- providing a crude sample to be subjected to detection analysis;- diluting said crude sample using a processing buffer comprising a protein-digesting enzyme, wherein the crude sample is diluted up to a protein concentration ranging from 0.1f xg/f xl to 10µg/µl, wherein said protein-digesting enzyme is proteinase K and is comprised between 1 mg/mL and 1[j, g/mL in said processing buffer, said processing buffer comprising TrisHCl buffer solution, said TrisHCl buffer solution being comprised between 1 M and 10 mM and the pH being between 7.0 and 10.0;- treating the diluted crude sample by performing a ramp of increasing and then decreasing temperature;- providing a PCR reaction mixture comprising two or more pairs of amplification primers for amplifying in a multiplex approach two or more target nucleic acids and an amplification buffer comprising an intercalating molecule or compound incorporated into the double-stranded amplicon and emitting a detectable signal;- performing PCR amplification using said PCR reaction mixture and said treated diluted crude sample;- performing, at the end of the PCR amplification, an HRM analysis on the PCR reaction mixture and said treated diluted crude sample previously subjected to PCR amplification; wherein the method further comprises monitoring, during the HRM analysis, the change in the signal emission resulting from the temperature-induced denaturation of the double-stranded amplicons into two single-stranded DNA, due to the release of the intercalating molecule or compound, wherein the method further comprises detecting DNA targets in the crude sample, through a reader analysing the signal variation and obtaining the result of the analysis through a graphic interface connected to said reader, wherein treating the diluted crude sample by performing a ramp of increasing and then decreasing temperature comprises performing the following temperature steps:- a first incubation step ranging from 5 to 60 minutes in a temperature range between 25°C and 70°C,- a second incubation step ranging from 5 to 60 minutes in a temperature range between 90°C and 100°C,- a third incubation step ranging from 5 to 60 minutes in a temperature range between 0°C and 10°C.

The DNA target is a HPV pathogen DNA target.

According to possible embodiments, the crude sample can be collected with a dedicated biological specimen collector, and possibly the processing buffer can be embedded inside the collector.

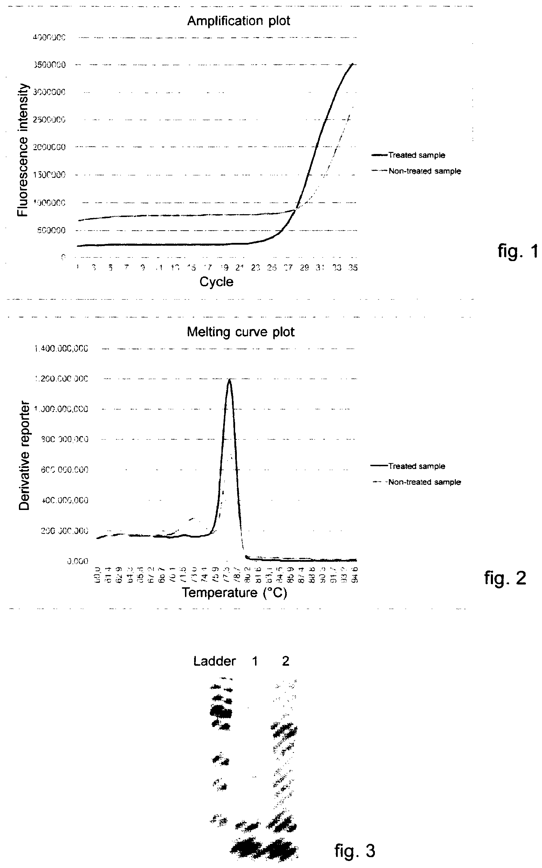

The complete pre-treatment with the processing buffer is crucial to obtain good amplification plots, decreasing the fluorescence background of the amplification signal and increasing fluorescence intensity of HRM analysis melting peaks; these are fundamental features that lead to an increased diagnostic sensitivity, specificity and accuracy of the analysis. In particular, in the case where the intercalating molecule or compound is an intercalating fluorescent dye, the initial high fluorescence background measured in the absence of processing buffer treatment is due to the complexity of the biological matrix, where proteins (mucins in case of mucus) together with other biological components entrap fluorescent dyes leading to a high fluorescent background. The treatment with the processing buffer according to the present disclosure has a pivotal role reducing the biological complexity of crude samples and avoiding structures able to retain fluorescent dyes that interfere with the PCR reaction and with the HRM analysis. The graph of Figure 1 shows the effect of the buffer P treatment on the final PCR amplification reaction. The grey amplification plot shows high initial fluorescence background compared to the treated sample that is represented with the black curve. The graph of Figure 2 shows the effect of the processing buffer treatment on the final HRM genotyping analysis. The grey melting curve has a lower fluorescence melting peak compared to the treated sample represented with the black curve.

Different patterns of protein contents of vaginal mucus were observed by SDS-PAGE gel analysis followed by Coomassie staining (see Figure 3); lane 1 shows the effect on vaginal mucus treated with the processing buffer according to the present disclosure in using a multistep temperature ramps. Lane 2 contains non-treated vaginal mucus. This panel shows that the pre-treatment with the processing buffer according to the present disclosure can reduce the biological complexity of crude sample (in this case vaginal mucus) affecting the protein matrix of the sample.

According to possible embodiments, the biological collector used to collect the crude sample, for instance vaginal or cervical mucus, can be suspended in the processing buffer according to the present disclosure, in order to achieve correct and required sample dilution.

According to the present disclosure, the final dilution of the crude sample, obtained for instance from the resuspension of biological collector, is crucial for the results of the analysis. As above discussed, in the final dilution, protein concentration advantageously ranges from 0.1 (µg/µl to 10 µg/µl.

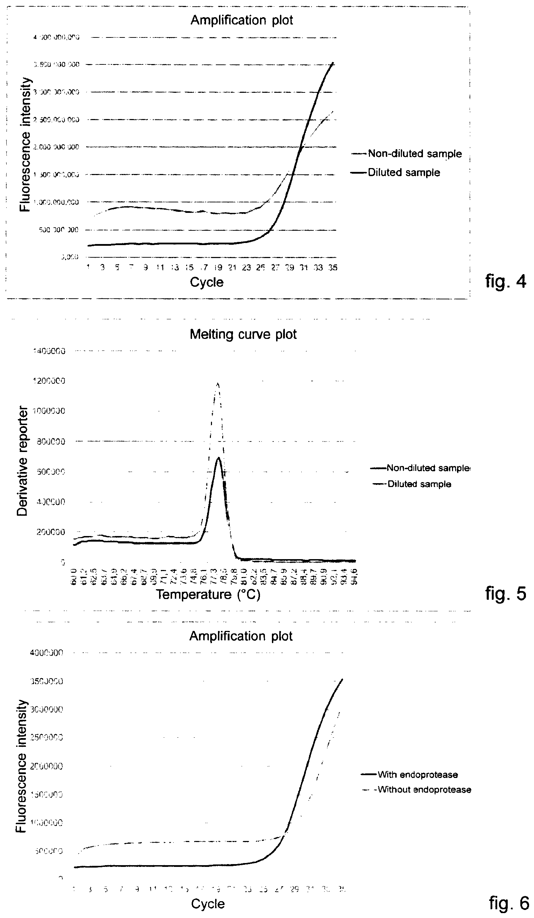

The graph of Figure 4 shows the effect of the crude samples dilution on the final PCR amplification reaction. The grey amplification plot (non-diluted sample) has a high initial fluorescence background compared to the correct diluted sample according to the present disclosure that is represented through the black curve.

The graph of Figure 5 shows the effect of the crude samples dilution on the final HRM genotyping analysis. The black melting curve (non-diluted sample) has a lower fluorescence melting peak compared to the correct diluted sample represented with the grey curve.

According to the present disclosure, the treatment of diluted crude sample occurs in the processing buffer containing at least the protein-digesting enzyme, and possibly a hydrolase, a pH stabilizer, a preservative, performing a ramp of temperature through a multistep process, wherein the temperature is first increased and then decreased. In particular, first the diluted crude sample in the processing buffer is heated, for example in a double step heating, and then it is cooled, for example in a single step cooling.

In possible implementations, a heating/cooling device can be used, configured for subjecting the diluted crude sample to a ramp of increasing and then decreasing temperature. As will be described more in detail herein below, the heating/cooling device can be a PCR amplification device or machine, a thermocycler, a thermocycler used for PCR reaction or the like embodying, including or embedding such heating/cooling device. In possible implementations, cooling can be obtained, for instance, by using specific Peltier cells or modules. According to the invention the ramp of temperature includes:
- a first incubation step ranging from 5 to 60 minutes in a temperature range between 25°C and 70°C, preferably between 50° and 60° C,
- a second incubation step ranging from 5 to 60 minutes in a temperature ranging from 90°C to 100°C,
- a third incubation step ranging from 5 to 60 minutes in a temperature ranging from 0°C to 10°C.

Advantageously, the protein-digesting enzyme contained in the processing buffer is crucial to obtain an efficient PCR amplification and a clear melting analysis, avoiding an excess of fluorescence background that can affect the sensitivity, specificity and accuracy of the final result.

The graph of Figure 6 shows the effect of protein-digesting enzyme, e.g. endoprotease, on the final PCR amplification plot. The grey amplification plot (processing buffer without endoprotease) has a higher fluorescence background compared to the sample treated with the processing buffer including endoprotease represented by the black curve.

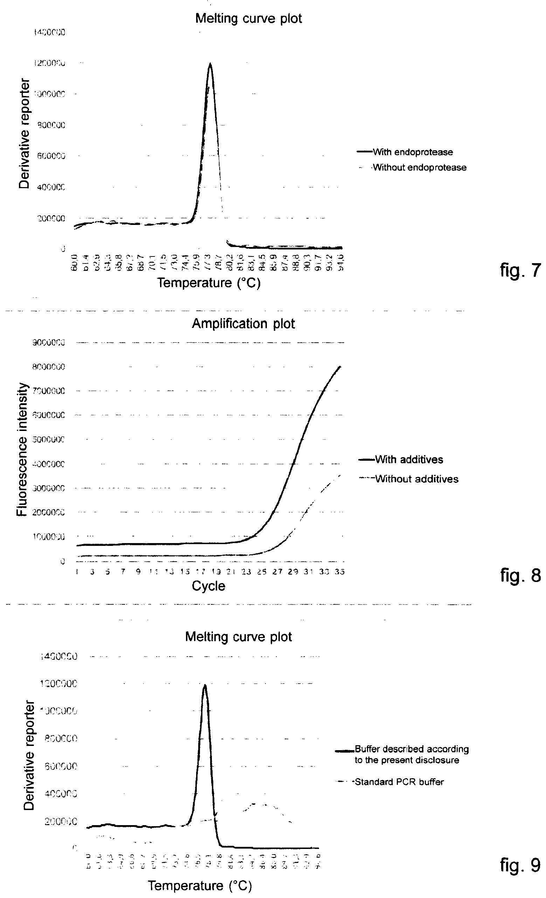

The graph of Figure 7 shows the effect of protein-digesting enzyme, e.g. endoprotease, on the final HRM genotyping analysis. The grey melting curve (processing buffer without endoprotease) has a lower fluorescence melting peak compared to the sample treated with the processing buffer including endoprotease that is represented by the black curve.

According to embodiments, combinable with all embodiments described herein, performing PCR amplification using said PCR reaction mixture and said treated diluted crude sample includes amplifying the purified target nucleic acid using said PCR reaction mixture to generate an amplicon or amplification product.

According to embodiments, combinable with all embodiments described herein, the amplification primers are sufficiently complementary to the target nucleic acid to hybridize therewith and trigger polymerase-mediated synthesis.

According to embodiments, combinable with all embodiments described herein, the PCR reaction mixture comprises the amplification primers and the amplification buffer.

According to embodiments, the amplification buffer is comprised in a diagnostic kit that is part of the present disclosure.

In one embodiment, combinable with all embodiments described herein, the PCR reaction mixture further comprises a DNA polymerase. The DNA polymerase is comprised in said amplification buffer. The DNA polymerase is an enzyme that polymerizes new DNA strands. For instance, heat resistant or heat stable polymerase can be used, since it is more likely to remain intact during the high-temperature DNA denaturation process. One example of heat resistant or heat stable polymerase that can be used in embodiments described herein is taq polymerase. Moreover, the polymerase that can be used in association with embodiments described herein is a hot-start polymerase. In a possible implementation, the hot-start polymerase can be (Hot Start) @Taq DNA Euroclone, (Hot Start) Phire Thermo Scientific, (Hot Start) Phusion Thermo Scientific, or (Hot Start) Gold Taq polymerase Sigma.

In one further embodiment, combinable with all embodiments described herein, the PCR reaction mixture may further comprise deoxynucleoside triphosphates (dNTPs) or analogues. dNTPs or analogues are comprised in said amplification buffer. dNTPs or analogues are used to provide the building blocks from which the DNA polymerase synthetizes a new DNA strand. dNTPs can be substituted by functional analogues like adenine, cytosine, guanine, tymine, uracil, orotidine, inositate, xanthylate, or others.

As above described, the PCR reaction mixture comprises said intercalating molecule or compound, being incorporated into the double-stranded amplicon or amplification product and emitting fluorescence or any other detectable signal. The intercalating molecule or compound can be comprised in said amplification buffer.

In particular, according to embodiments, the intercalating molecule can be any sensor or reporter molecule emitting a signal that can be detected by a reader analysing an electric signal variation in terms of inductance, current, electric potential, in case of conductimetric, amperometric, voltammetric detection, or the presence of light at specific wavelengths in case of a fluorescence/chemiluminescence detection, or light scattering and/or refraction/diffraction phenomena, in case of a plasmonic optical detection.

For instance, in some implementations the intercalating molecule or compound can be an intercalating dye emitting fluorescence. According to possible implementations, specific DNA intercalating dye, at a final concentration range from 1 to 8 µM, can be one or more of the following dyes: SYTO-9, SYTO-13, SYTO-16, SYTO-64, SYTO-82, YO-PRO-1, SYTO-60, SYTO-62, TOTO-3, POPO-3, BOBO-3, doxorubicinconjugated quantum dot nanoparticles or similar.

In yet another embodiment, combinable with all embodiments described herein, the PCR reaction mixture may further comprise a source of monovalent or bivalent cations. The source of monovalent or bivalent cations is comprised in said amplification buffer. For example, a chloride containing monovalent ion or bivalent ions can be used. As a source of monovalent cations, potassium ions can be used. K⁺ can be obtained from potassium salts, e.g. potassium chloride, in particular potassium chloride at a concentration of 0.1 M. As source of bivalent cations magnesium or manganese ions can be used. Mg²⁺ can be obtained from magnesium salts, e.g. magnesium chloride.

In one further embodiment, combinable with all embodiments described herein, the PCR reaction mixture may further comprise bovine serum albumin (BSA). The BSA is comprised in said amplification buffer.

In one further embodiment, combinable with all embodiments described herein, the PCR reaction mixture further comprises one or more detergents. In possible implementations, said detergent can be Nonidet-P40 at a concentration of about 0.5%.

In still another embodiment, combinable with all embodiments described herein, the PCR reaction mixture may further comprise additives. The additives can be comprised in some embodiments of the above-mentioned amplification buffer. In possible implementations, the additives that can be used are selected between one, more or all of additives in a group comprising: NP40, DMSO, TMAC (Tetramethylammonium Chloride), Acetamide, Triton, Formamide, Betaine, *E. Coli* ssDNA binding protein, Glycerol, L-Camitine and Gelatin. Advantageously, in embodiments exploiting a fluorescence detection, the presence of additives can be important to avoid a high basal fluorescence background allowing an increased diagnostic sensitivity, specificity and accuracy (see Figures 1 and 8). In some possible implementations, the additives may further comprise a gelatin, for example at a concentration of about 0.1%. In yet further possible implementations, the additives may further comprise an enhancer. For example, the enhancer can be L-Camitin at a concentration of about 0,42M. In still further possible implementations, the additives may further comprise sugar alcohol, for example sorbitol at a concentration of about 25mM.

The use of additives according to embodiments described herein is advantageous to avoid a high basal fluorescence background in the PCR reaction, allowing an increased diagnostic sensitivity, specificity and accuracy. The graph of Figure 8 shows the effect of the additives according to possible embodiments described herein, on the final PCR amplification plot. The black amplification plot (PCR amplification buffer without additives) has a higher initial fluorescence background compared to amplification performed with PCR amplification buffer with additives (grey curve).

According to embodiments, combinable with all embodiments described herein, amplifying the purified target nucleic acid using said PCR reaction mixture to generate an amplicon or amplification product includes thermocycling by performing a ramp of temperature steps. In one possible implementation, a ramp of temperature includes performing the following temperature steps:
- denaturation ranging from 95° to 98°C ranging from 1 to 30 seconds;
- annealing in a ranging from 50°C to 70°C ranging from 1 to 60 seconds;
- extension in a range between 60°C and 75°C ranging from 0 second to 5 minutes.

In possible implementations, the number of cycles of thermocycling is of at least 30 cycles, for instance between 30 and 50 cycles. One possible example is 35 cycles.

In one possible implementation, hot-start polymerase can be used. Hot-start PCR avoids non-specific amplification of DNA by inactivating the polymerase at lower temperatures, for instance through antibodies interaction, chemical modification or aptamer technology. Typically, a specific inhibitor, such as an aptamer-based inhibitor or specific antibodies can be used to block the polymerase at lower temperatures. If hot-start polymerase is used, an initial incubation step which ranges from 95°C to 98°C from 1 second to 10 minutes is performed. This initial incubation step is necessary for activation of polymerase.

According to embodiments, combinable with all embodiments described herein, the method includes, during thermocycling in the PCR amplification, performing said monitoring the change in the signal, e.g. fluorescence, emission resulting from the temperature-induced denaturation of the double-stranded amplicons or amplification products into two single-stranded DNA, due to the release of the intercalating molecule or compound, i.e. intercalating dye. The intercalating molecule or compound binds to DNA in the double-strand configuration. At each amplification cycle, amplicons are generated in which the intercalating molecule or compound binds in the extension step, during which the signal (e.g. fluorescence) is acquired.

Advantageously, the PCR reaction can occur in a real-time PCR machine, that allows monitoring the change in the signal, e.g. fluorescence, emission at each amplification cycle in the PCR amplification, in turn allowing quantification of the presence of amplicons and quantification, therefore, of the viral DNA in the amplification phase.

In other embodiments, the PCR amplification may occur in a thermocycling machine able to acquire said signal emission each 0.1°C/second or less.

According to embodiments, combinable with all embodiments described herein, performing the HRM analysis includes performing a ramp of temperature on the PCR reaction mixture previously subjected to PCR amplification. In one possible implementation, a ramp of temperature includes performing the following temperature steps:
- incubation at 95°C ranging from 1 seconds to 60 seconds
- incubation at 60°C ranging from 1 seconds to 2 minutes
- ramping up to 95°C increasing the temperature 0.1°C/second or less, and performing said monitoring the change in the signal, e.g. fluorescence, emission resulting from the temperature-induced denaturation of the double-stranded amplicons or amplification products into two single-stranded DNA, due to the release of the intercalating molecule or compound, i.e. intercalating dye.

The method according to the present disclosure as above described allows to genotype different pathogens through HRM analysis amplifying DNA directly from crude samples. Conversely, conventional PCR systems and methods do not allow to obtain clear melting curves starting without DNA extraction. The graph of Figure 9 shows a comparison between the melting curves obtained from the method according to the present disclosure (black curve) and a standard PCR buffer of the prior art used to perform PCR directly from crude samples, without treatment with the processing buffer (grey curve). The black melting curve enables to clearly discriminate a single melting peak allowing to easily genotype a pathogen. The grey line is obtained using a standard direct PCR system; it is possible to notice that the fluorescence background is very high and it is not present a single, clear and high melting peak.

According to embodiments, combinable with all embodiments described herein, PCR amplification can be for instance performed in a PCR thermocycler. Such PCR thermocycler can embody, include or integrate the heating/cooling device for performing the treatment of the crude sample with the above described processing buffer.

According to further embodiments, combinable with all embodiments described herein, PCR amplification can be typically performed in a real-time PCR machine.

Since, according to the present disclosure, the same sample is first subjected to the PCR amplification and then to HRM analysis, the whole method can be performed in a single apparatus, in particular a real-time PCR machine.

According to possible embodiments, the PCR amplification and detection can be performed simultaneously by means of Real Time PCR in any setup known in the art, including quantitative Real time PCR allowing assessment of the pathogenic load in the infected sample, followed by HRM analysis, performed in the same real-time PCR machine.

However, in other embodiments, the two operations, i.e. PCR amplification and HRM analysis, can also be performed in separate and distinct apparatuses coupled or associated each other, for instance a typical thermocycler for the PCR amplification and then a real-time PCR configured for HRM analysis.

For example, in possible implementations, the detection can be performed using a dedicated PCR device, also in portable format, containing a specific Peltier module coupled with a fluorescence optical reader or other appropriate reading device, able to perform HRM analysis.

In still further implementations, the detection via the PCR amplification can be performed using a dedicated PCR device containing for instance a specific Peltier module coupled to a read-out device different than a fluorescent read-out device, for instance a chemiluminescent or electrochemical read-out device, a conductimetric, amperometric, voltammetric read-out device, plasmonic optical red-out device or any other suitable read-out device.

According to possible embodiments, the PCR reaction can be performed using a container, e.g. a test tube or vial, possibly provided with a closure cap, or a well of a microplate, inside which the crude sample can be contained. In possible implementations, the crude sample can be treated using the above described processing buffer prior to be disposed inside said container, or alternatively the crude sample can be directly disposed into the container used for the PCR reaction and treated therein with the above processing buffer.

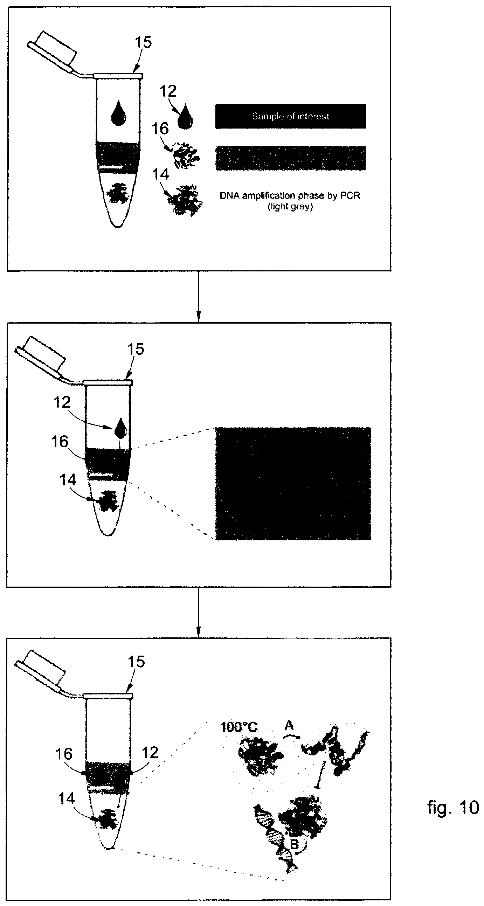

In still further embodiments described using Figures 10 and 11, which are combinable with all embodiments described herein, the processing buffer and the PCR mixture or amplification buffer can be arranged in different regions of the container used for the PCR reaction or arranged split in compartments of such container, so that the crude sample is diluted and treated therein using the processing buffer according to the present disclosure, and then subjected to PCR amplification using the same container, in a combined method essentially involving a first step of sample treatment with protein-digesting enzyme and a second step of PCR amplification.

For instance, the processing buffer and the PCR mixture or amplification buffer can be characterized by different states of matter and disposed directly inside the container used for the PCR reaction.

In particular, in possible embodiments the container can comprise a first phase and a second phase, or upper phase.

In Figure 10 numeral 12 denotes the sample of interest, numeral 14 denotes the first phase containing DNA polymerase, numeral 15 denotes the container and numeral 16 denotes the second phase containing the processing buffer.

As depicted in Figure 10, upper panel, the first phase 14 can be localized at the bottom of the container 15 and can be solid, semi-solid or gel, i.e. jellified. The PCR mixture or amplification buffer is comprised in said first phase 14.

In this embodiment, the solid/semi-solid/jellified state of the first phase 14 can be obtained by using a polysaccharide polymer material, such as agarose, in layer or as a diluent, and including the polymerase therein; or by using an air-bubble layer. In alternative, the polymerase can be embedded in microbeads, such as agarose microbeads or alginate microbeads.

The second phase, or upper phase, 16 is disposed on or above the first phase 14 and is liquid, e.g. aqueous. The processing buffer is comprised in said second phase 16.

In these embodiments, therefore, the pre-treatment using the processing buffer, the PCR reaction followed by the HRM analysis occurs in the same container 15. Advantageously, in possible implementations such three operations can be performed using the same specific container 15 according to the following protocol, involving the first step of sample treatment with protein-digesting enzyme and the second step of PCR amplification:
- first incubation step ranging from 5 to 60 minutes in a range of temperature between 50°C and 70°C;
- second incubation step ranging from 5 to 60 minutes in a range of temperature between 90°C and 100°C;
- third incubation step ranging from 5 to 60 minutes in a range of temperature between 0°C and 10°C;
- incubation at a temperature from 95°C to 98°C ranging from 5 second to 10 minutes (if hot-start polymerase is used);
- thermocycling, e.g. at least 30 cycles of the following steps:
   - denaturation at 95-98°C ranging from 5 to 30 seconds;
   - annealing in a range between 50°C and 70°C ranging from 1 to 60 seconds;
   - extension in a range between 70°C and 75°C ranging from 1 second to 5 minutes;
- incubation at 95°C ranging from 5 seconds to 60 seconds;
- incubation at 60°C ranging from 30 seconds to 2 minutes;
- ramping up to 95°C increasing the temperature 0.1°C/second or less.

According to such embodiment, the PCR mixture or amplification buffer of the first phase 14 present in the container 15 is advantageously protected during the pre-treatment with the second phase 16 comprising the processing buffer, and successively the content of the PCR mixture or amplification buffer is released at the end of the pre-treatment thanks to state changes of the two phases due to heating as above described.

In particular, as shown in Figure 10, second panel, in a first step the sample 12 is loaded into the container 15 and thus subjected to treatment with the processing buffer of the second phase 16. In the first step of the combined method, in the liquid second phase 16 the proteins of the sample 12 are degraded by the protein-digesting enzyme, e.g. endoprotease, (indicated by letter A) to a whole protein sample (indicated by letter C), in order to release the DNA template (indicated by letter B). The temperature of the first incubation ranging from 50°C to 70°C, e.g. 56°C, is advantageous and optimal for maximum processivity (i.e the ability of the enzyme to catalyse consecutive reactions without releasing its substrate) of the proteolytic enzyme. In the meanwhile, the DNA polymerase, inside the solid/semi-solid/gel first phase 14, is protected from degradation.

In the second step, as shown in Figure 10 lower panel, increasing the temperature ranging from 95 to 98°C, e.g. in the case of incubation for hot-start polymerase activation, results in the denaturation of the protein-digesting enzyme. In the meanwhile, the first phase 12 turns from solid/semi-solid/gel phase into a liquid phase due to temperature increase and, for example, the hot-start polymerase can be activated. Denaturation of the protein-digesting enzyme at such temperature avoids polymerase degradation (A) and activates the polymerase that amplifies the DNA template by PCR reaction protocol (B).

Embodiments described herein can be used for diagnostic purposes. In particular, in the following, specific ranges of the reagents present in the processing buffer and in the two possible implementations of the PCR reaction mixture are described, that can be used for diagnostic purposes. Subsequently, specific ranges are described that can be used for specific detection of the HPV DNA.

In one embodiment, the processing buffer for diagnostic purposes comprises proteinase K and TrisHCl buffer solution. For instance, one specific implementation of the processing buffer comprises:
a) proteinase K (between 1 mg/mL and 1 µg/mL);
b) TrisHCl buffer solution (between 1 M and 10 mM; pH between 7.0 and 10.0).

In one embodiment, a possible first amplification buffer for diagnostic purposes comprises the dNTPs, the source of mono or bivalent cations, the buffer solution, the BSA, the Hot Start DNA polymerase, the intercalating molecule or compound. For instance, one specific implementation of the first amplification buffer comprises:
a) dNTPs (final concentration range: from 0.05 mM to 0.3 mM)
b) MgCl₂ (final concentration range: from 0.3 mM to 4 mM)
c) TrisHCl buffer solution (final concentration range: from 10 mM to 50 mM; pH from 6.00 to 10.00)
d) KCl (final concentration range: from 10 mM to 50 mM)
e) BSA (final concentration range: from 0.005 to 0.05 mg/ml)
f) Hot Start polymerase
g) SYTO-9 (final concentration range: from 1µM to 8µM).

In another possible embodiment, a possible alternative second amplification buffer for diagnostic purposes is provided, that comprises the dNTPs, the source of mono or bivalent cations, the buffer solution, the BSA, the hot start DNA polymerase, the intercalating molecule or compound and the above-mentioned additives. By the addition of additives, the second amplification buffer can be used as a PCR enhancer buffer providing increased diagnostic sensitivity, specificity and accuracy as above discussed. For instance, one specific implementation of the alternative second amplification buffer comprises:
a) dNTPs (final concentration range: from 0.05 mM to 0.5 mM)
b) MgCl₂ (final concentration range: from 0.3 mM to 4 mM)
c) TrisHCl buffer solution (final concentration range: from 10 mM to 50 mM; pH from 6.00 to 10.00)
d) KCl (final concentration range: from 10 mM to 50 mM)
e) BSA (final concentration range: from 0.005 to 0.05 mg/ml)
f) Hot start polymerase
g) SYTO-9 (final concentration range: from 1µM to 8 µM)
h) TMAC (Tetramethylammonium Chloride) (final concentration range: from 10mM to 100 mM)
i) Acetamide (final concentration range: from 0% to 5%)
j) Formamide (final concentration range: from 0% to 5%)
k) Betaine (final concentration range: from 0 µM to 5M)
l) Gelatin (final concentration range: from 0.01 mg/ml to 1 mg/ml).

In the embodiments of methods and diagnostic kit described herein for diagnostic purposes, since different PCR machine, i.e. different thermocycler models, may generate different curves and in particular different melting peaks, a calibrator is provided to set each real-time PCR machine for a correct and precise melting analysis required to genotype the pathogen.

Indeed, some variations might occur due to machine type, efficiency due to maintenance status or acquisition settings, the calibrator allows the adjustment of the observed measurements in a specific machine. The calibrator can be composed by synthetic oligonucleotides corresponding to the amplicons generated by the specific primers of the PCR reaction mixture according to the present disclosure. Advantageously, a machine-specific calibrator can be loaded in PCR runs periodically to check the effective melting temperature of the amplicons of a particular thermocycler machine, and comparing them with the expected melting temperature.

In the following, embodiments of methods and diagnostic kit according to the present disclosure are described for specific use for HPV diagnosis, using specific primers.

In one embodiment, methods and diagnostic kit of the present disclosure are used to detect Human Papillomavirus DNA in clinical samples and to discriminate different genotypes by HRM analysis.

In one embodiment, a specific processing buffer for HPV diagnosis, for example in the case of vaginal mucus sample, or PreservCyt-sample (or similar), or cervical swabs, or biopsies, formalin-fixed paraffin-embedded (*FFPE*) tissue or saliva, or urine or similar biological sample, comprises at least the following components:
- proteinase K (between 1 mg/mL and 1 µg/mL)
- TrisHCl buffer solution (between 1 M and 10 mM; pH from 7.0 to pH 10.0).

One example of possible specific concentration values of the reagents of this processing buffer for HPV diagnosis is the following:
- proteinase K at a final concentration of 500µg/mL;
- TrisHCl buffer solution at a final concentration of 150 mM and pH 9.0.

In one embodiment, a specific amplification buffer for HPV diagnosis comprises the following reagents with concentration expressed as ranges:
a) dNTPs (final concentration range: from 0.05 mM to 0.5 mM)
b) MgCl2 (final concentration range: from 0.3 mM to 4 mM)
c) TrisHCl buffer solution (final concentration range: from 10 mM to 50 mM; pH from 7.00 to 10.00)
d) KCl (final concentration range: from 10 mM to 50 mM)
e) BSA (final concentration range: from 0.005 mg/ml to 0.05 mg/ml)
f) Hot Start polymerase
g) SYTO-9 (final concentration range: from 1µM to 8µM).

One example of possible specific concentration values of the reagents is the following:
a) dNTPs (final concentration 0.2mM)
b) MgCl2 (final concentration 0.75mM)
c) TrisHCl buffer solution (final concentration 30mM: and pH 9.0)
d) KCl (final concentration 50mM)
e) Bovise Serum Albumin (final concentration 10µg/ml)
f) Hot start polymerase
g) SYTO-9 (final concentration 4µM).

In another possible embodiment, a further alternative possible specific amplification buffer for HPV diagnosis, providing increased diagnostic sensitivity, specificity and accuracy comprises the following reagents with concentration expressed as ranges:
a) dNTPs (final concentration range: from 0.05 mM to 0.3 mM)
b) MgCl2 (final concentration range: from 0.3 mM to 4 mM)
c) TrisHCl buffer solution (final concentration range: from 10 mM to 50 mM; pH from 7.00 to 10.00)
d) KCl (final concentration range: from 10 mM to 50 mM)
e) BSA (final concentration range: from 0.005 mg/ml to 0.05 mg/ml)
f) Hot start polymerase
g) SYTO-9 (final concentration range: from 1µM to 8µM)
h) TMAC (Tetramethylammonium Chloride) (final concentration range: from 10mM to 100mM)
i) Acetamide (final concentration range: from 0.5% to 5%)
j) Formamide (final concentration range: from 0.5% to 5%)
k) Betaine (final concentration range: from 100 µM to 5M)

1) Gelatin (final concentration range: from 0.01 mg/ml to 1 mg/ml).One example of possible specific concentration values of the reagents of this further alternative amplification buffer is the following:
   a) dNTPs (final concentration 0.15 mM)
   b) MgCl2 (final concentration 0.75 mM)
   c) TrisHCl buffer solution (final concentration 30mM; pH 9)
   d) KCl (final concentration: 40mM)
   e) BSA (final concentration 10µg/ml)
   f) Hot start polymerase
   g) SYTO-9 (final concentration 1 µM)
   h) TMAC (Tetramethylammonium Chloride, final concentration of 75 mM)
   i) Acetamide (final concentration 3%)
   j) Formamide (final concentration 1,5%)
   k) Betaine (final concentration 0,5M)
   l) Gelatin (final concentration 0,1mg/mL).

Advantageously, the diagnostic HPV test based on the embodiments described herein can provide at least an important diagnostic information, for example for HPV diagnostic, or other sexually transmissible diseases, like for instance Chlamydia infection, syphilis infection, or Gonorrhoea infection. For example, in the case of HPV diagnostic, the amplification curves obtained by the PCR allow the detection of the first 14 high risk genotypes (HPV 16, 18, 31, 33, 35, 39, 45, 51, 52, 56, 58, 59, 66, and 68 - Cit. IARC 2009) and thus give a diagnostic information. A sample is positive when the amplification curve occurs before 35 PCR cycles using a fluorescence threshold that range from 250.000 to 400.000. The present invention has been tested on positive samples (extracted DNA more than 200 HPV positive women with a CIN2+ cytology, according with Mejer guide lines, 2009). Clinical data showed that sensitivity of the diagnostic HPV test according to the embodiments described herein, intended as the sensitivity to detect High-Grade Squamous Intraepithelial Lesions (HSIL or CIN2/3 lesions, that are considered the clinical endpoint in the HPV diagnostics), is 98%. Sensitivity is the percentage of HPV-infected people with a diagnosed HSIL that are detected by the HPV test according to the embodiments described herein, divided by the total number of HPV-infected people with a diagnosed HSIL detected by conventional test (e.g. Pap test). The test specificity reaches almost the 100% and the test accuracy ranges from 0,93 to 0,98;

While the foregoing is directed to embodiments of the invention, other and further embodiments of the invention may be devised without departing from the basic scope thereof, and the scope thereof is determined by the claims that follow.

### BIBLIOGRAPHY

1. Munoz et al. HPV and the ethiology of human cancer. Vaccine (2006).
2. Herrero et al. Human Papillomavirus and oral cancer: The International Agency for Research on Cancer multicentre study. J Natl Cancer (2003).
3. de Villiers et al., Classification of Papillomavirus. Virology (2004).
4. Munoz et al., Epidemiologic classification of Human Papillomavirus Types associated with cervical cancer. N Engl J Med (2003).
5. Cuzick et al., Overview of the European and North American studies on HPV testing in primary cervical cancer screening. Int J Cancer (2006).
6. Clavel et al. Human papillomavirus testing in primary screening for the detection of high-grade cervical lesions: a study of 7932 women. Br. J. Cancer (2001).
7. Ronco et al. Efficacy of human papillomavirus testing for the detection of invasive cervical cancers and cervical intraepithelial neoplasia: a randomized controlled trial. Lancet Oncol. (2010).
8. Arbyn et al. Evidence regarding human papillomavirus testing in secondary prevention of cervical cancer. Vaccine (2012).
9. Dillner et al. Long term predictive values of cytology and human papillomavirus testing in cervical cancer screening: joint European cohort study. BMJ (2008).
10. Söderlund-Strand et al. Genotyping of human papillomavirus in triaging of lowgrade cervical cytology. Am J Obstet Gynecol (2011).
11. Naucler et al. HPV type-specific risks of high-grade CIN during 4 years of followup: a population-based prospective study. Br. J. Cancer (2007).
12. Pierce Campbell et al. Long-term persistence of oral human papillomavirus type 16: the HPV Infection in Men (HIM) study. Cancer Prev Res (Phila) (2015).
13. Donà et al. Incidence, clearance and duration of cutaneous beta and gamma human papillomavirus anal infection. J Infect Oct (2016).
14. Dillner et al. Translational mini-review series on vaccines: monitoring of human papillomavirus vaccination. Clin. Exp.Immunol. (2007).
15. Poljak et al. Commercially available molecular tests for human papillomaviruses (HPV): 2015 update. J Clin Vir (2015).

## Claims

1. A method for pathogen DNA targets detection directly in crude samples through Polymerase Chain Reaction (PCR) and genotyping via High Resolution Melting (HRM) analysis, said pathogen being human papilloma virus (HPV), said method comprising:
- providing a crude sample to be subjected to detection analysis;
- diluting said crude sample using a processing buffer comprising a protein-digesting enzyme, wherein the crude sample is diluted up to a protein concentration ranging from 0.1µg/µl to 10µg/µl, wherein said protein-digesting enzyme is proteinase K and is comprised between 1 mg/mL and 1 µg/mL in said processing buffer, said processing buffer comprising TrisHCl buffer solution, said TrisHCl buffer solution being comprised between 1 M and 10 mM and the pH being between 7.0 and 10.0;
- treating the diluted crude sample by performing a ramp of increasing and then decreasing temperature;
- providing a PCR reaction mixture comprising two or more pairs of amplification primers for amplifying in a multiplex approach two or more target nucleic acids and an amplification buffer comprising an intercalating molecule or compound incorporated into the double-stranded amplicon and emitting a detectable signal;
- performing PCR amplification using said PCR reaction mixture and said treated diluted crude sample;
- performing, at the end of the PCR amplification, an HRM analysis on the PCR reaction mixture and said treated diluted crude sample previously subjected to PCR amplification;
wherein the method further comprises monitoring, during the HRM analysis, the change in the signal emission resulting from the temperature-induced denaturation of the double-stranded amplicons into two single-stranded DNA, due to the release of the intercalating molecule or compound,
wherein the method further comprises detecting DNA targets in the crude sample, through a reader analysing the signal variation and obtaining the result of the analysis through a graphic interface connected to said reader, wherein treating the diluted crude sample by performing a ramp of increasing and then decreasing temperature comprises performing the following temperature steps:
- a first incubation step ranging from 5 to 60 minutes in a temperature range between 25°C and 70°C,
- a second incubation step ranging from 5 to 60 minutes in a temperature range between 90°C and 100°C,
- a third incubation step ranging from 5 to 60 minutes in a temperature range between 0°C and 10°C.

2. The method according to claim 1, wherein said processing buffer further comprises one or more of a hydrolase, a pH stabilizer, a preservative.

3. The method according to claims 1 or 2, wherein the amplification buffer of the PCR reaction mixture further comprises additives selected between one, more or all of additives in a group comprising: NP40, DMSO, TMAC (Tetramethylammonium Chloride), Acetamide, Triton, Formamide, Betaine, *E. Coli* ssDNA binding protein, Glycerol, L-Camitine and Gelatin

4. The method according to any of claims 1 to 3, wherein a container (15) for performing the PCR reaction is provided, said crude sample being contained in said container (15), wherein a first region of said container (15) contains a first phase (14) comprising said PCR mixture or amplification buffer, said first phase (14) being solid, semi-solid or gel, further wherein a second region of said container (15), provided on or above said first region, contains a second, or upper, phase (16) comprising said processing buffer, said second, or upper, phase (16) being liquid, wherein diluting said crude sample using said processing buffer, and treating the diluted crude sample by performing a ramp of temperature occur in the second region and the PCR reaction followed by the HRM analysis occur, in the same said container (15), in the first region.

5. The method according to any of claims 1 to 4, wherein the PCR amplification is a real-time PCR, or wherein said PCR amplification is performed in a thermocycler or in a real-time PCR thermocycler, wherein, when the PCR amplification is performed in a thermocycler, the HRM analysis is performed in a separate real-time PCR thermocycler, while when the PCR amplification is performed in a real-time PCR thermocycler, the HRM analysis is performed in the same real-time PCR thermocycler.

6. A diagnostic kit for pathogen DNA targets detection directly in crude samples through Polymerase Chain Reaction (PCR) and genotyping via High Resolution Melting (HRM) analysis, said pathogen being human papilloma virus (HPV), wherein said kit comprises a processing buffer and a Polymerase Chain Reaction (PCR) reaction mixture used for performing PCR amplification and a subsequent HRM analysis on the PCR reaction mixture previously subjected to real-time PCR, wherein said processing buffer comprises a protein-digesting enzyme, wherein said protein-digesting enzyme is proteinase K and is comprised between 1 mg/mL and 1 µg/mL in said processing buffer, said processing buffer comprising TrisHCl buffer solution, said TrisHCl buffer solution being comprised between 1 M and 10 mM and the pH being between 7.0 and 10.0, and further wherein said PCR reaction mixture comprises two or more pairs of amplification primers for amplifying in a multiplex approach two or more target nucleic acids and an amplification buffer comprising an intercalating molecule or compound incorporated into the double-stranded amplicon and emitting a detectable signal.

7. The diagnostic kit according to claim 6, wherein said processing buffer further comprises one or more of a hydrolase, a pH stabilizer, a preservative.

8. The diagnostic kit according to claim 6 or 7, wherein said amplification buffer further comprises additives selected between one, more or all of additives in a group comprising: NP40, DMSO, TMAC (Tetramethylammonium Chloride), Acetamide, Triton, Formamide, Betaine, *E. Coli* ssDNA binding protein, Glycerol, L-Carnitine and Gelatin.

9. The diagnostic kit according to any of claims 6 to 8, wherein said diagnostic kit comprises a container (15) for performing the PCR reaction, said container (15) being suitable to contain said crude sample, wherein a first region of said container (15) contains a first phase (14) comprising said PCR mixture or amplification buffer, said first phase (14) being solid, semi-solid or gel, further wherein a second region of said container (15), provided on or above said first region, contains a second, or upper, phase (16) comprising said processing buffer, said second, or upper, phase (16) being liquid.

10. An apparatus for performing pathogen DNA targets detection directly in crude samples through Polymerase Chain Reaction (PCR) and genotyping via HRM analysis, said pathogen being human papilloma virus (HPV), wherein said apparatus comprises:
- a processing buffer comprising a protein-digesting enzyme, for diluting said crude sample, wherein said protein-digesting enzyme is proteinase K and is comprised between 1 mg/mL and 1 µg/mL in said processing buffer, said processing buffer comprising TrisHCl buffer solution, said TrisHCl buffer solution being comprised between 1 M and 10 mM and the pH being between 7.0 and 10.0;
- a heating/cooling device configured for subjecting the diluted crude sample to a treatment according to a ramp of increasing and then decreasing temperature, comprising performing the following temperature steps:
- a first incubation step ranging from 5 to 60 minutes in a temperature range between 25°C and 70°C,
- a second incubation step ranging from 5 to 60 minutes in a temperature range between 90°C and 100°C,
- a third incubation step ranging from 5 to 60 minutes in a temperature range between 0°C and 10°C;
- a PCR reaction mixture comprising two or more pairs of amplification primers to amplify through multiplex approach two or more nucleic acids targets and an amplification buffer comprising an intercalating molecule or compound incorporated into the double-stranded amplicon and emitting a detectable signal;
- a PCR amplification device configured for using said PCR reaction mixture and for subjecting the treated diluted crude sample to a PCR amplification;
- a device for performing an HRM analysis on the PCR reaction mixture previously subjected to PCR amplification;
wherein the PCR reaction mixture comprises two or more pairs of amplification primers for amplifying in a multiplex approach two or more target nucleic acids;
- monitoring means for monitoring, during the HRM analysis, the change in the signal emission resulting from the temperature-induced denaturation of the double-stranded amplicons into two single-stranded DNA, due to the release of the intercalating molecule or compound,
- a reader analysing the signal variation for detecting DNA targets in the crude sample, so that the result of the analysis can be obtained through a graphic interface connected to said reader.

## Patentansprüche

1. Verfahren zur Detektion von Pathogen-DNA-Targets, direkt in Rohproben, durch Polymerase-Kettenreaktion (PCR) und Genotypisierung mittels High-Resolution-Melting (HRM)-Analyse, wobei das Pathogen das humane Papillomavirus (HPV) ist und das Verfahren aufweist:
- Bereitstellen einer Rohprobe, die einer Detektionsanalyse unterzogen werden soll;
- Verdünnen der Rohprobe unter Verwendung eines Verarbeitungspuffers, der ein eiweißspaltendes Enzym aufweist, wobei die Rohprobe bis zu einer Proteinkonzentration im Bereich von 0,1 µg/µl bis 10 µg/µl verdünnt wird, wobei das eiweißspaltende Enzym Proteinase K ist und in dem Verarbeitungspuffer in einer Menge zwischen 1 mg/ml und 1 µg/ml enthalten ist, der Verarbeitungspuffer TrisHCl-Pufferlösung aufweist, die TrisHCl-Pufferlösung in einer Menge zwischen 1 M und 10 mM enthalten ist und der pH-Wert zwischen 7,0 und 10,0 liegt;
- Behandeln der verdünnten Rohprobe durch Durchführen einer Temperaturrampe mit ansteigender und dann abfallender Temperatur;
- Bereitstellen einer PCR-Reaktionsmischung aufweisend zwei oder mehr Paare von Amplifikationsprimern zur Amplifikation von zwei oder mehr Targetnukleinsäuren in einem Multiplex-Ansatz und einen Amplifikationspuffer aufweisend ein interkalierendes Molekül oder eine interkalierende Verbindung, das bzw. die in das doppelsträngige Amplikon eingebaut ist und ein detektierbares Signal emittiert;
- Durchführen von PCR-Amplifikation unter Verwendung der PCR-Reaktionsmischung und der behandelten, verdünnten Rohprobe;
- Durchführen, am Ende der PCR-Amplifikation, einer HRM-Analyse der PCR-Reaktionsmischung und der behandelten, verdünnten Rohprobe, die zuvor der PCR-Amplifikation unterzogen wurde;
wobei das Verfahren ferner das Beobachten der Änderung der Signalemission während der HRM-Analyse aufweist, die sich aus der temperaturinduzierten Denaturierung des doppelsträngigen Amplicons in zwei einzelsträngige DNA ergibt, aufgrund der Freisetzung des interkalierenden Moleküls oder der interkalierenden Verbindung,
wobei das Verfahren ferner die Detektion von DNA-Targets in der Rohprobe durch ein Lesegerät, das die Signaländerung analysiert und das Erhalten des Ergebnisses der Analyse durch eine mit dem Lesegerät verbundene, grafische Schnittstelle aufweist, wobei die Behandlung der verdünnten Rohprobe durch Durchführen einer Temperaturrampe mit ansteigender und dann abfallender Temperatur die folgenden Temperaturschritte aufweist:
- einen ersten Inkubationsschritt von 5 bis 60 Minuten in einem Temperaturbereich zwischen 25°C und 70°C,
- einen zweiten Inkubationsschritt von 5 bis 60 Minuten in einem Temperaturbereich zwischen 90°C und 100°C,
- einen dritten Inkubationsschritt von 5 bis 60 Minuten in einem Temperaturbereich zwischen 0°C und 10°C.

2. Verfahren gemäß Anspruch 1, wobei der Verarbeitungspuffer ferner eine oder mehrere von einer Hydrolase, einem pH-Stabilisator, einem Konservierungsmittel aufweist.

3. Verfahren gemäß Anspruch 1 oder 2, wobei der Amplifikationspuffer der PCR-Reaktionsmischung ferner Additive aufweist, die ausgewählt sind aus einem, mehreren oder allen Additiven in einer Gruppe aufweisend: NP40, DMSO, TMAC (Tetramethylammoniumchlorid), Acetamid, Triton, Formamid, Betain, *E. Coli* ssDNA bindendes Protein, Glycerin, L-Carnitin und Gelatine.

4. Verfahren gemäß einem der Ansprüche 1 bis 3, wobei ein Behälter (15) zur Durchführung der PCR-Reaktion bereitgestellt wird und die Rohprobe in dem Behälter (15) enthalten ist, wobei ein erster Bereich des Behälters (15) eine erste Phase (14) enthält, die das PCR-Gemisch oder den Amplifikationspuffer aufweist und die erste Phase (14) fest, halbfest oder ein Gel ist, wobei ferner ein zweiter Bereich des Behälters (15), der auf oder über dem ersten Bereich vorgesehen ist, eine zweite oder obere Phase (16) enthält, die den Verarbeitungspuffer aufweist und die zweite oder obere Phase (16) flüssig ist, wobei das Verdünnen der Rohprobe unter Verwendung des Verarbeitungspuffers und das Behandeln der verdünnten Rohprobe durch Durchführen einer Temperaturrampe in dem zweiten Bereich und die PCR-Reaktion, gefolgt von der HRM-Analyse, in demselben Behälter (15), in dem ersten Bereich stattfinden.

5. Verfahren gemäß einem der Ansprüche 1 bis 4, wobei die PCR-Amplifikation eine Echtzeit-PCR ist, oder wobei die PCR-Amplifikation in einem Thermocycler oder in einem Echtzeit-PCR-Thermocycler durchgeführt wird, wobei, wenn die PCR-Amplifikation in einem Thermocycler durchgeführt wird, die HRM-Analyse in einem separaten Echtzeit-PCR-Thermocycler durchgeführt wird, während, wenn die PCR-Amplifikation in einem Echtzeit-PCR-Thermocycler durchgeführt wird, die HRM-Analyse in demselben Echtzeit-PCR-Thermocycler durchgeführt wird.

6. Diagnostik-Kit zur Detektion von Pathogen-DNA-Targets direkt in Rohproben durch Polymerase-Kettenreaktion (PCR) und Genotypisierung mittels High Resolution Melting (HRM)-Analyse, wobei das Pathogen das humane Papillomavirus (HPV) ist und das Kit aufweist: einen Verarbeitungspuffer und eine Polymerase-Kettenreaktion (PCR) Reaktionsmischung zur Durchführung von PCR-Amplifikation und einer anschließenden HRM-Analyse der PCR-Reaktionsmischung, die zuvor einer Echtzeit-PCR unterzogen wurde, wobei der Verarbeitungspuffer ein eiweißspaltendes Enzym aufweist, wobei das eiweißspaltende Enzym Proteinase K ist und in dem Verarbeitungspuffer in einer Menge zwischen 1 mg/ml und 1 µg/ml enthalten ist, der Verarbeitungspuffer TrisHCl-Pufferlösung aufweist, die TrisHCl-Pufferlösung in einer Menge zwischen 1 M und 10 mM enthalten ist und der pH-Wert zwischen 7,0 und 10,0 liegt; und wobei ferner die PCR-Reaktionsmischung aufweist zwei oder mehr Paare von Amplifikationsprimern zur Amplifikation von zwei oder mehr Targetnukleinsäuren in einem Multiplex-Ansatz und einen Amplifikationspuffer aufweisend ein interkalierendes Molekül oder eine interkalierende Verbindung, das bzw. die in das doppelsträngige Amplikon eingebaut ist und ein detektierbares Signal emittiert.

7. Diagnostik-Kit gemäß Anspruch 6, wobei der Verarbeitungspuffer ferner eine oder mehrere von einer Hydrolase, einem pH-Stabilisator, einem Konservierungsmittel aufweist.

8. Diagnostik-Kit gemäß Anspruch 6 oder 7, wobei der Amplifikationspuffer ferner Additive aufweist, die ausgewählt sind aus einem, mehreren oder allen Additiven in einer Gruppe aufweisend: NP40, DMSO, TMAC (Tetramethylammoniumchlorid), Acetamid, Triton, Formamid, Betain, *E. Coli* ssDNA bindendes Protein, Glycerin, L-Carnitin und Gelatine.

9. Diagnose-Kit gemäß einem der Ansprüche 6 bis 8, wobei das Diagnose-Kit einen Behälter (15) zur Durchführung der PCR-Reaktion aufweist, wobei der Behälter (15) geeignet ist die Rohprobe zu enthalten, wobei ein erster Bereich des Behälters (15) eine erste Phase (14) enthält, die das PCR-Gemisch oder den Amplifikationspuffer aufweist und die erste Phase (14) fest, halbfest oder ein Gel ist, wobei ferner ein zweiter Bereich des Behälters (15), der auf oder über dem ersten Bereich vorgesehen ist, eine zweite oder obere Phase (16) enthält, die den Verarbeitungspuffer aufweist und die zweite oder obere Phase (16) flüssig ist.

10. Vorrichtung zur Durchführung von Pathogen-DNA-Target Detektion direkt in Rohproben durch Polymerase-Kettenreaktion (PCR) und Genotypisierung mittels HRM-Analyse, wobei das Pathogen ein humanes Papillomavirus (HPV) ist und die Vorrichtung aufweist:
- einen Verarbeitungspuffer zum Verdünnen der Rohprobe, der ein eiweißspaltendes Enzym aufweist, wobei das eiweißspaltende Enzym Proteinase K ist und in dem Verarbeitungspuffer in einer Menge zwischen 1 mg/ml und 1 µg/ml enthalten ist, der Verarbeitungspuffer TrisHCl-Pufferlösung aufweist, die TrisHCl-Pufferlösung in einer Menge zwischen 1 M und 10 mM enthalten ist und der pH-Wert zwischen 7,0 und 10,0 liegt;
- eine Heiz-/Kühlvorrichtung, die geeignet ist, die verdünnte Rohprobe einer Behandlung gemäß einer Temperaturrampe mit ansteigender und dann abfallender Temperatur zu unterwerfen, die folgenden Temperaturschritte aufweist:
- einen ersten Inkubationsschritt von 5 bis 60 Minuten in einem Temperaturbereich zwischen 25°C und 70°C,
- einen zweiten Inkubationsschritt von 5 bis 60 Minuten in einem Temperaturbereich zwischen 90°C und 100°C,
- einen dritten Inkubationsschritt von 5 bis 60 Minuten in einem Temperaturbereich zwischen 0°C und 10°C.
- eine PCR-Reaktionsmischung aufweisend zwei oder mehr Paare von Amplifikationsprimern zur Amplifikation von zwei oder mehr Targetnukleinsäuren in einem Multiplex-Ansatz und einen Amplifikationspuffer aufweisend ein interkalierendes Molekül oder eine interkalierende Verbindung, das bzw. die in das doppelsträngige Amplikon eingebaut ist und ein detektierbares Signal emittiert;
- eine PCR-Amplifikationsvorrichtung, die geeignet ist die PCR-Reaktionsmischung zu verwenden und die behandelte, verdünnte Rohprobe einer PCR-Amplifikation zu unterziehen;
- eine Vorrichtung zur Durchführung einer HRM-Analyse an der PCR-Reaktionsmischung, die zuvor PCR-Amplifikation unterzogen wurde;
wobei die PCR-Reaktionsmischung zwei oder mehr Paare von Amplifikationsprimern aufweist, um in einem Multiplex-Ansatz zwei oder mehr Targetnukleinsäuren zu amplifizieren;
- Beobachtungsmittel zur Beobachtung der Änderung der Signalemission während der HRM-Analyse, die sich aus der temperaturbedingten Denaturierung des doppelsträngigen Amplikons in zwei einzelsträngige DNA aufgrund der Freisetzung des interkalierenden Moleküls oder der interkalierenden Verbindung ergibt,
- ein Lesegerät, das die Signaländerung zur Detektion von DNA-Targets in der Rohprobe analysiert, so dass das Ergebnis der Analyse über eine mit dem Lesegerät verbundene grafische Schnittstelle erhalten werden kann.

## Revendications

1. Procédé pour la détection de cibles d'ADN pathogènes directement dans des échantillons bruts par réaction en chaîne par polymérase (PCR) et génotypage par analyse de fusion à haute résolution (HRM), ledit pathogène étant le virus du papillome humain (HPV), ledit procédé comprenant les étapes consistant à :
- fournir un échantillon brut à soumettre à une analyse de détection ;
- diluer ledit échantillon brut à l'aide d'un tampon de traitement comprenant une enzyme digérant les protéines, dans lequel l'échantillon brut est dilué jusqu'à une concentration en protéines dans la plage de 0,1 µg/µl à 10 µg/µl, dans lequel ladite enzyme digérant les protéines est la protéinase K et est comprise dans la plage de 1 mg/mL à 1 µg/mL dans ledit tampon de traitement, ledit tampon de traitement comprenant une solution tampon de TrisHCl, ladite solution tampon de TrisHCl étant dans la plage de 1 M à 10 mM et le pH étant dans la plage de 7,0 à 10,0 ;
- traiter l'échantillon brut dilué en le faisant passer par une rampe d'augmentation puis de diminution de température ;
- fournir un mélange réactionnel de PCR comprenant deux paires d'amorces d'amplification ou plus pour amplifier dans une approche multiplex deux acides nucléiques cibles ou plus et un tampon d'amplification comprenant une molécule ou un composé d'intercalation incorporé(e) dans l'amplicon bicaténaire et émettant un signal détectable ;
- effectuer une amplification par PCR en utilisant ledit mélange réactionnel de PCR et ledit échantillon brut dilué traité ;
- effectuer, à la fin de l'amplification par PCR, une analyse HRM sur le mélange réactionnel de PCR et ledit échantillon brut dilué traité préalablement soumis à l'amplification par PCR ;
dans lequel le procédé comprend en outre la surveillance, pendant l'analyse HRM, de la variation de l'émission de signal résultant de la dénaturation induite par la température des amplicons bicaténaires en deux ADN monocaténaires, en raison de la libération de la molécule ou du composé d'intercalation,
dans lequel le procédé comprend en outre la détection de cibles d'ADN dans l'échantillon brut, par l'intermédiaire d'un lecteur analysant une variation de signal et obtenant le résultat de l'analyse par l'intermédiaire d'une interface graphique connectée audit lecteur, dans lequel le traitement de l'échantillon brut dilué par la réalisation d'une rampe d'augmentation puis de diminution de température comprend la réalisation des étapes de température suivantes :
- une première étape d'incubation allant de 5 à 60 minutes dans une plage de température comprise entre 25°C et 70°C,
- une deuxième étape d'incubation allant de 5 à 60 minutes dans une plage de température comprise entre 90°C et 100°C,
- une troisième étape d'incubation allant de 5 à 60 minutes dans une plage de température comprise entre 0°C et 10°C.

2. Procédé selon la revendication 1, dans lequel ledit tampon de traitement comprend en outre un ou plusieurs parmi une hydrolase, un stabilisateur de pH, un conservateur.

3. Procédé selon la revendication 1 ou 2, dans lequel le tampon d'amplification du mélange réactionnel de PCR comprend en outre des additifs choisis parmi un, plusieurs ou tous les additifs dans un groupe comprenant : NP40, DMSO, TMAC (Chlorure de tétraméthylammonium), Acétamide, Triton, Formamide, Bétaïne, protéine de liaison d'ADNsb *E. Coli,* Glycérol, L-Carnitine et Gélatine.

4. Procédé selon l'une quelconque des revendications 1 à 3, dans lequel un contenant (15) pour effectuer la réaction de PCR est prévu, ledit échantillon brut étant contenu dans ledit contenant (15), dans lequel une première région dudit contenant (15) contient une première phase (14) comprenant ledit mélange de PCR ou tampon d'amplification, ladite première phase (14) étant solide, semi-solide ou en gel, dans lequel en outre une seconde région dudit contenant (15), prévue sur ou au-dessus de ladite première région, contient une seconde phase, ou phase supérieure (16) comprenant ledit tampon de traitement, ladite seconde phase, ou phase supérieure (16) étant liquide, dans lequel la dilution dudit échantillon brut en utilisant ledit tampon de traitement, et le traitement de l'échantillon brut dilué en effectuant une rampe de température se produit dans la seconde région et la réaction de PCR suivie par l'analyse HRM se produisent, dans ledit même contenant (15), dans la première région.

5. Procédé selon l'une quelconque des revendications 1 à 4, dans lequel l'amplification par PCR est une PCR en temps réel, ou dans lequel ladite amplification par PCR est effectuée dans un thermocycleur ou dans un thermocycleur de PCR en temps réel, dans lequel, lorsque l'amplification par PCR est effectuée dans un thermocycleur, l'analyse HRM est effectuée dans un thermocycleur de PCR en temps réel séparé, tandis que lorsque l'amplification par PCR est effectuée dans un thermocycleur de PCR en temps réel, l'analyse HRM est effectuée dans le même thermocycleur de PCR en temps réel.

6. Kit de diagnostic pour une détection de cibles d'ADN pathogènes directement dans des échantillons bruts par réaction en chaîne par polymérase (PCR) et par génotypage par une analyse de fusion à haute résolution (HRM), ledit pathogène étant le virus du papillome humain (HPV), dans lequel ledit kit comprend un tampon de traitement et un mélange réactionnel de réaction en chaîne par polymérase (PCR) utilisés pour effectuer une amplification par PCR et une analyse HRM consécutive sur le mélange réactionnel de PCR préalablement soumis à une PCR en temps réel, dans lequel ledit tampon de traitement comprend une enzyme digérant les protéines, dans lequel ledit l'enzyme digérant les protéines est la protéinase K et est dans la plage de 1 mg/mL à 1 µg/mL dans ledit tampon de traitement, ledit tampon de traitement comprenant une solution tampon de TrisHCl, ladite solution tampon TrisHCl étant dans la plage de 1 M à 10 mM et le pH étant dans la plage de 7,0 à 10,0, et en outre dans lequel ledit mélange réactionnel de PCR comprend deux paires d'amorces d'amplification ou plus pour amplifier dans une approche multiplex deux acides nucléiques cibles ou plus et un tampon d'amplification comprenant une molécule ou un composé d'intercalation incorporé(e) dans l'amplicon bicaténaire et émettant un signal détectable.

7. Kit de diagnostic selon la revendication 6, dans lequel ledit tampon de traitement comprend en outre une ou plusieurs parmi une hydrolase, un stabilisateur de pH, un conservateur.

8. Kit de diagnostic selon la revendication 6 ou 7, dans lequel ledit tampon d'amplification comprend en outre des additifs choisis parmi un, plusieurs ou tous les additifs dans un groupe comprenant : NP40, DMSO, TMAC (Chlorure de tétraméthylammonium), Acétamide, Triton, Formamide, Bétaïne, protéine de liaison d'ADNsb *E*. *Coli,* Glycérol, L-Camitine et Gélatine.

9. Kit de diagnostic selon l'une quelconque des revendications 6 à 8, dans lequel ledit kit de diagnostic comprend un contenant (15) pour effectuer la réaction de PCR, ledit contenant (15) étant approprié pour contenir ledit échantillon brut, dans lequel une première région dudit contenant (15) contient une première phase (14) comprenant ledit mélange de PCR ou tampon d'amplification, ladite première phase (14) étant solide, semi-solide ou en gel, dans lequel en outre une seconde région dudit contenant (15), prévue sur ou au-dessus de ladite première région, contient une seconde phase, ou phase supérieure (16) comprenant ledit tampon de traitement, ladite seconde phase, ou phase supérieure (16) étant liquide.

10. Appareil pour effectuer une détection de cibles d'ADN pathogènes directement dans des échantillons bruts par réaction en chaîne par polymérase (PCR) et génotypage par analyse HRM, ledit pathogène étant le virus du papillome humain (HPV), dans lequel ledit appareil comprend :
- un tampon de traitement comprenant une enzyme digérant les protéines, pour diluer ledit échantillon brut, dans lequel ladite enzyme digérant les protéines est la protéinase K et est dans la plage de 1 mg/ml à 1 µg/ml dans ledit tampon de traitement, ledit tampon de traitement comprenant une solution tampon de TrisHCl, ladite solution tampon de TrisHCl étant dans la plage de 1 M à 10 mM et le pH étant compris entre 7,0 et 10,0 ;
- un dispositif de chauffage/refroidissement configuré pour soumettre l'échantillon brut dilué à un traitement selon une rampe d'augmentation puis de diminution de température, comprenant la réalisation des étapes de température suivantes :
- une première étape d'incubation allant de 5 à 60 minutes dans une plage de température comprise entre 25°C et 70°C,
- une deuxième étape d'incubation allant de 5 à 60 minutes dans une plage de température comprise entre 90°C et 100°C,
- une troisième étape d'incubation allant de 5 à 60 minutes dans une plage de température comprise entre 0°C et 10°C ;
- un mélange réactionnel de PCR comprenant deux paires d'amorces d'amplification ou plus pour amplifier par approche multiplex deux cibles d'acide nucléique ou plus et un tampon d'amplification comprenant une molécule ou un composé d'intercalation incorporé(e) dans l'amplicon bicaténaire et émettant un signal détectable ;
- un dispositif d'amplification par PCR configuré pour utiliser ledit mélange réactionnel de PCR et pour soumettre l'échantillon brut dilué traité à une amplification par PCR ;
- un dispositif pour effectuer une analyse HRM sur le mélange réactionnel de PCR préalablement soumis à une amplification par PCR ;
dans lequel le mélange réactionnel de PCR comprend deux paires d'amorces d'amplification ou plus pour amplifier dans une approche multiplex deux acides nucléiques cibles ou plus ;
- des moyens de surveillance pour surveiller, au cours de l'analyse HRM, la modification de l'émission de signal résultant de la dénaturation induite par la température des amplicons bicaténaires en deux ADN monocaténaires, du fait de la libération de la molécule ou du composé d'intercalation,
- un lecteur analysant la variation de signal pour détecter des cibles d'ADN dans l'échantillon brut, de sorte que le résultat de l'analyse peut être obtenu par l'intermédiaire d'une interface graphique connectée audit lecteur.
